(19) Europäisches Patentamt
European Patent Office
Office européen des brevets

(11) **EP 3 801 760 B1**

(12) **EUROPEAN PATENT SPECIFICATION**

(45) Date of publication and mention
of the grant of the patent:
**12.01.2022 Bulletin 2022/02**

(51) Int Cl.:
***A61N 5/10*** (2006.01)

(21) Application number: **19723808.2**

(86) International application number:
**PCT/EP2019/062753**

(22) Date of filing: **17.05.2019**

(87) International publication number:
**WO 2019/228824 (05.12.2019 Gazette 2019/49)**

(54) **DOSE ERROR DETERMINATION DEVICE**

DOSISFEHLERBESTIMMUNGSVORRICHTUNG

DISPOSITIF DE DÉTERMINATION D'ERREUR DE DOSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.05.2018 EP 18174501**

(43) Date of publication of application:
**14.04.2021 Bulletin 2021/15**

(73) Proprietor: **Koninklijke Philips N.V.
5656 AG Eindhoven (NL)**

(72) Inventors:
• **BONDAR, Maria, Luiza**
**5656 AE Eindhoven (NL)**
• **ISOLA, Alfonso, Agatino**
**5656 AE Eindhoven (NL)**
• **BAL, Matthieu, Frédéric**
**5656 AE Eindhoven (NL)**

(74) Representative: **Philips Intellectual Property &
Standards
High Tech Campus 52
5656 AG Eindhoven (NL)**

(56) References cited:
**EP-A1- 2 808 057      US-A1- 2014 163 302
US-A1- 2016 059 039      US-A1- 2016 140 300
US-A1- 2016 166 855**

• **NEIL KIRBY ET AL: "An automated deformable
image registration evaluation of confidence tool",
PHYSICS IN MEDICINE AND BIOLOGY,
INSTITUTE OF PHYSICS PUBLISHING, BRISTOL
GB, vol. 61, no. 8, 30 March 2016 (2016-03-30) ,
XP020303406, ISSN: 0031-9155, DOI:
10.1088/0031-9155/61/8/N203 [retrieved on
2016-03-30]**

**Description**

FIELD OF THE INVENTION

The present invention relates to a dose error determination device for forecasting radiation dose error for a body volume comprising one or more regions of interest subject to radiation treatment based on a dose plan.

BACKGROUND OF THE INVENTION

In clinical practice, decision for treatment adaptation is typically taken by evaluating the original treatment plan on the daily patient anatomy. The evaluation can be performed automatically by using deformable image registration (DIR) between the planning image and a volumetric daily-acquired image of the patient such as, for example, magnetic resonance image, ultrasound image, computed tomography image, or cone beam computed tomography image. DIR automatically propagates the contours of the regions of interest (ROIs) from a planning computer tomography image to a daily image and enables automated assessment of the radiation therapy plan, and of the radiotherapy dose delivered to the ROIs without the need for manual delineations. A treatment plan is calculated based on dosimetric constraints for each ROI such as, for example, minimum therapeutic dose level for the tumor, and maximum tolerated dose levels for organs at risk. Treatment plan evaluation on the current anatomy recalculates the dose parameters and updates a scorecard. The scorecard indicates whether the current values of the dosimetric parameters fulfill the dosimetric constraints. The recalculated dose parameters and the updated score card information are used to estimate the need for treatment adaptation.

The article "An automated deformable image registration evaluation of confidence tool" by Neil Kirby et al., Physics in Medicine and Biology, volume 61, pages N203 to N214 (2016) criticizes DIR for potentially producing errors, and in that its accuracy is not a fixed quantity, but varies on a case-by-case basis. As a solution to these issues, Kirby et al. describe an automated program to create a patient- and voxel-specific evaluation of DIR accuracy. A software tool is described to perform this evaluation for the application of a clinical DIR algorithm to a set of patient images. The software tool uses algorithms to generate deformations and applies them to these images along with processing, to generate sets of test images, with known deformations that are similar to the actual ones and with realistic noise properties. A clinical DIR algorithm is applied to these test image sets. From these tests, the software tool generates spatial and dose uncertainty estimates for each image voxel based on a Student's t distribution. In the reported study, four commercially available DIR algorithms were used to deform a dose distribution associated with a virtual pelvic phantom image set, and the software tool was used to generate dose uncertainty estimates for each deformation.

SUMMARY OF THE INVENTION

It is an object of the present invention to provide a dose error determination device that requires a comparatively low processing expenditure for providing assistance in estimating the impact of any type of spatial error on a dose value of a predetermined dose plan for one or more regions of interest in clinical applications.

According to a first aspect of the present invention, a dose error determination device for forecasting radiation dose error for a body volume comprising one or more regions of interest subject to radiation treatment based on a dose plan is described. The dose error determination device comprises:

- a dose plan unit configured to provide dose plan data indicative of dose values associated with voxels of a current image of the body volume comprising voxels associated with the region of interest;
- an input unit configured to request and receive user input indicative of a value of a spatial-error quantity corresponding to an amount of a spatial error of voxel coordinates in the current image associated with corresponding features of the body volume;
- a dose-error determining unit, which is configured:
- to generate, using the dose plan data for the respective region of interest and the value of the spatial-error quantity, dose variation data indicative of a rate of change of the dose values in a respective modified region of interest that in comparison with the respective region of interest is expanded in volume by an amount corresponding with the value of the spatial-error quantity; and
- to determine and provide a respective value of a dose-error quantity associated with the respective region of interest, using a calculation rule representing a positive correlation of the dose-error quantity with the determined dose variation data; and
- an output unit, which receives the respective value of the dose-error quantity, and is configured to provide an output indicative thereof.

The dose error determination device of the present invention is based on the recognition that the described known solution by Kirby et al. for determining DIR-related dose error is based on the assumptions that artificially produced registration errors are of the same magnitude as the real unknown registration error of the tested algorithm and that the artificially produced registration errors are statistically independent and Gaussian distributed. These assumptions are normally not applicable to real patient data in clinical applications. For example, the method by Kirby et al. could produce errors of lower magnitude than the real errors, thus misleading the user on the correctness of the tested algorithm.

Furthermore, the present invention recognizes that the solution proposed by Kirby et al. does not allow a user investigating the robustness of dose volume parameters for spatial errors of a given magnitude.

Furthermore, the method proposed by Kirby et al. requires the implementation and the use of several deformable registration algorithms, which can be time consuming and is therefore not feasible for real clinical applications.

These and other issues are overcome by the dose error determination device of the first aspect of the present invention.

The dose error determination device of the first aspect of the present invention is for forecasting radiation dose error for a body volume comprising one or more regions of interest subject to radiation treatment based on a dose plan. The device has a dose plan unit providing dose plan data indicative of dose values associated with voxels of a current image of the body volume comprising voxels associated with the region of interest. In operation of the device, an input unit user input indicative of a value of a spatial-error quantity corresponding to an amount of a spatial error of voxel coordinates in the current image associated with corresponding features of the body volume

The dose error determination device provides a respective value of a dose-error quantity associated with the respective region of interest, using a calculation rule representing a positive correlation of the dose-error quantity with determined dose variation data. The dose variation data is indicative of a rate of change of the dose values in a respective modified region of interest that in comparison with the respective region of interest is expanded in volume by an amount corresponding with the value of the spatial-error quantity. Such dose variation data is generated by the dose-error determining unit using the dose plan data for the respective region of interest and a value of the spatial-error quantity provided by user-input via the input unit of the dose error determination device. The input value of the spatial-error quantity is used by the dose-error determining unit.

The dose error determination device thus takes account of the recognition that sources of said spatial error of voxel coordinates are manifold. They include, for instance, but are not limited to, missing voxels, extra voxels, or displacement of the ROI's within the body volume. Other examples of spatial errors are caused by a low resolution of the current image, yet other examples by an incorrect spatial definition of the regions of interest. Thus, among the manifold causes for spatial error, the error component contributed by the deformable image registration may have less relevance.

The dose-error determining unit therefore receives the dose plan data from the dose plan unit and the value of the spatial-error quantity by user input via the input unit and is configured to generate the dose variation data and the dose-error quantity for one or more given regions of interest. The dose variation data is indicative of a rate of change of the dose value in a respective modified region of interest which, in comparison with the respective region of interest, is expanded in volume by an amount corresponding to the value of the spatial-error quantity. The determination of the dose-error quantity is performed using a calculation rule that represents a positive correlation of the dose-error quantity with the determined dose variation data. A positive correlation means that, in accordance with the calculation rule used by the dose-error determining unit, for any increase in value of the dose variation data a resulting value of the dose error-quantity also increases, and that for any decrease in value of the dose variation data, the resulting dose error-quantity also decreases.

The dose error determination device also includes an output unit which receives the respective value of the dose-error quantity associated to one of the one or more regions of interest and provides an output indicative thereof.

The provision of an output indicative of a value of the dose-error quantity that is associated to a respective region of interest can be therefore used by the user to efficiently estimate or forecast a potential dose-error in dependence on the input value of the spatial-error quantity, which allows efficiently estimating the dose error irrespective of particular causes for spatial error.

In the following, embodiments of the dose error determination device of the first aspect of the invention will be described.

In a particular embodiment, the value of the spatial-error quantity is provided as a number indicative of an amount of a spatial error of voxel coordinates that has been determined by the user based on differences between positions of the regions of interest in a current image and in an initial image of the body volume. For instance, radiation dose plans are generated based on the initial image of the body volume obtained by imaging methods having capable of providing high resolution images such as, for example, computed tomography (CT). The current patient anatomy can differ from the patient anatomy at the time when the treatment plan was generated, for instance, due to a size increase or to a size reduction of a given region of interest. Also, the current patient anatomy may be imaged by using another imaging technique, including, but not limited to, magnetic resonance, ultrasound, or cone beam computed tomography. Differences in image resolution between imaging methods may also result in differences in the patient anatomy between the initial image on which the treatment plan is based and the current image of the body volume. The present embodiment thus requires a particularly low processing expenditure for determining the value of the spatial-error quantity. The value of the spatial-error quantity should preferably be indicative of a maximum spatial error of voxel coordinates, i.e. of a maximal spatial distance between a given point or feature in a region of interest on the initial image and the respective point or feature on the current image.

In another embodiment, the dose error determination device additionally comprises a statistics unit that stores statistical population data. The statistical population data associates statistical values associated with the spatial-error quantity to respective regions of interest. The association is based on a statistical population of previous measurements. In this embodiment, the input unit is configured to request and receive user input indicative of at least one region of interest and, in response to receiving such user input, to access the statistics unit and to ascertain the statistical values associated with the spatial-error quantity for those regions of interest indicated by the user input, and to subsequently provide the request for user input indicative of the value of the spatial-error quantity in association with an output indicative of the ascertained respective statistical value. The statistics unit and the corresponding input unit of this particular embodiment may be included in any of the embodiments discussed in the following.

In another embodiment of the dose error determination device the dose-error determining unit is configured to determine, for each voxel of the modified region of interest, local dose variation data representing a product of an amount of a local rate of change of the current dose values associated with the given voxel and of the value of the spatial-error quantity, and to determine the dose variation data associated with the region of interest as a maximum amount of the product within the modified region of interest. Thus, for a given region of interest, the dose variation data provided by the dose-determining unit is the maximum value of the determined local dose variation data. The local dose variation data is obtained, in a particular embodiment, by performing a high order discretization of a continuous Nabla operator that defines the spatial variation of the dose values on a computational grid.

In another embodiment, the dose-error determining unit is additionally configured to identify a maximum dose value among the dose plan data, and, upon determining that the identified maximum dose value is smaller in amount than the determined maximum of the product within the modified region of interest, the dose-error determining unit is configured to provide to the output unit the identified maximum dose value as the respective value of the dose-error quantity associated with the respective region of interest. This offers a realistic output since the maximum possible output value is limited to the highest dose value of the dose plan. This can be relevant for particular combinations of dose plan data and spatial-error quantity, where a product of the amount of a local rate of change of the current dose values associated with the given voxel and of the value of the spatial-error quantity results in value a local dose variation data that is higher than the maximal dose value of the dose plan data.

Another embodiment in accordance with the first aspect of the invention comprises a dose-error determining unit that is additionally or alternatively configured to determine, as the dose variation quantity, a maximum dose value and a minimum dose value among those dose values corresponding to voxels located within a distance given by the spatial-error quantity with respect to any voxel associated with each region of interest. The maximum dose value and the minimum dose value provide can advantageously be used by a user to estimate worst-case scenario dose error.

In another embodiment of the dose-error determination device of the invention, the input unit is additionally configured to request and receive dose-tolerance user input indicative of a tolerance interval of the dose-error quantity associated with a given region of interest, the dose-error determining unit is further configured to receive the tolerance interval, and to determine, additionally using the tolerance interval, a relation between the determined value of the dose-error quantity and the tolerance interval associated with the given region of interest. In this embodiment, the output unit is configured to provide the output as additionally being indicative of the determined relation. This embodiment enables the user to assign tolerance intervals of the dose-error quantity to one or more regions of interest. Depending on the region of interest, on the treatment plan, or on the individual patient subject to the radiation treatment, different quantities of dose-error can be tolerated without necessarily having to adapt an original treatment plan. A user can thus assign comparatively lower tolerance intervals to those regions of interest that are more critical to the treatment plan, or close to regions of the patient that should not be exposed to high levels of radiation. The output provided by the output unit takes into account the determined relation.

Another embodiment of the dose determination device comprises a dose-objective input interface for receiving dose-objective data for the region of interest. The dose-objective data is indicative of desired values of dose-objective quantities associated with the previous dose plan statistics unit. In this embodiment, the dose-error determining unit is additionally configured, using the dose-plan data and the determined value of the dose-error quantity for the region of interest, to generate dose-forecast data for the region of interest indicative of forecast values of the dose-objective quantities. Further, the output unit of this embodiment is configured to provide the output as additionally being indicative of the dose-objective data and of the dose-forecast data.

Another embodiment of the dose determination device of the first aspect comprises a ROI modelling unit. The ROI modelling unit is configured to generate, in response to a selection instruction received via the input unit, second instance region of interest data. The second instance region of interest data is data associated with a given one of the one or more regions of interest by modifying, in accordance with a selected one of a set of one or more respective uncertainty models defining spatial coordinate transformation instructions for transforming the region of interest only in regard to its spatial voxel coordinates, thus providing a representation of a transformed region of interest. In this embodiment, the dose-error determining unit is additionally configured to determine and provide an expected value of the dose-error quantity associated with the transformed region of interest. Further, the input unit is additionally configured to request and receive user input indicative of the uncertainty model to be used by the ROI modelling unit.

In a variant of this embodiment the output unit is additionally configured to receive two-dimensional image data representing an image of the respective region of interest, and to generate and provide the output of the dose-error quantity in the form of a graphical overlay onto the image of the respective region of interest.

In another embodiment of the dose error determination device, the output unit is alternatively or additionally configured to associate a color-code value to the received value of the dose-error quantity, in accordance with a predetermined color code assigning different colors to different predetermined value intervals of the dose-error quantity. It is also configured to generate and provide the output as additionally being indicative of the color assigned to the respective value of the dose-error quantity

In another embodiment of the dose error determination device, the dose plan unit is additionally configured to provide a plurality of dose plans, each comprising respective dose plan data associated with alternative dose plans for the region of interest. In this particular embodiment, the dose-error determining unit is further configured to provide plan-robustness data indicative of a respective plan-robustness value for each dose plan and in dependence of the received value of the spatial-error quantity, the plan-robustness value being determined in accordance with a predetermined rule based on the determined values of the dose-error quantities associated with the one or more regions of interest. Further, the output unit of this embodiment is further configured to receive the plan-robustness data and to provide an output indicative thereof.

In one of these embodiments, different dose plans are compared in terms of robustness to spatial errors of a given ROI by the dose determining unit. Such dose determining unit is advantageously configured to use a plurality of values of the spatial-error quantity and to determine, for each of the values of the spatial error quantity and for each of the dose plans provided a respective value of the dose error quantity. One particular embodiment provides the value of the dose error quantity as a standard deviation value of a fractional ROI volume that is expected to receive a radiation dose higher than a threshold dose value. Alternatively, other embodiment outputs the respective value of the dose error quantity as a width value of a dose volume histogram for a given radiation dose value. The respective width values of the different dose plans at the given dose value can be output as numerical quantities.

A second aspect of the present invention is a computer-implemented dose error determination method for forecasting radiation dose error for a body volume comprising one or more regions of interest subject to radiation treatment based on a dose plan. The dose error determination method comprises:

- providing dose plan data indicative of dose values associated with voxels of a current image of the body volume comprising voxels associated with the region of interest;
- requesting and receiving user input indicative of a value of a spatial-error quantity corresponding to an amount of a spatial error of voxel coordinates in the current image associated with corresponding features of the body volume;
- generating, using the dose plan data for the respective region of interest and the value of the spatial-error quantity, dose variation data indicative of a rate of change of the dose values in a respective modified region of interest that in comparison with the respective region of interest is expanded in volume by an amount corresponding with the value of the spatial-error quantity;

- determining and providing a respective value of a dose-error quantity associated with the respective region of interest, using a calculation rule representing a positive correlation of the dose-error quantity with the determined dose variation data; and
- providing an output indicative the respective value of the dose-error quantity.

The method of the second aspect of the invention shares the advantages of the dose error determination device of the first aspect of the invention.

Embodiments of the method of the second aspect of the invention have additional method features corresponding to the additional features of embodiments of the dose error determination device described hereinabove.

According to a third aspect of the invention, a computer program is provided, which comprises instructions which, when the program is executed by a computer, cause the computer to carry out the method of the second aspect of the invention or one of its embodiments.

It shall thus be understood that the dose error determination device of claim 1, the dose error determination method of claim 12 and the computer program of claim 13 have corresponding embodiments. Furthermore, it shall be understood that a preferred embodiment of the present invention can also be formed by any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows a schematic representation of an embodiment of a dose error determination device.
Fig. 2A shows a first image of a body volume that includes a region of interest and which is divided in voxels, each of which is associated to a radiation dose value.
Fig. 2B shows a second image of the body volume that includes the region of interest showing a different shape when compared to that of the first image.
Fig. 3 shows an example of an informative table displayed by a display unit used in combination with a dose error determination device.
Fig. 4 shows an example of a sensitivity map displayed by a display unit used in combination with a dose error determination device.
Fig. 5 shows an example of a graph plotting values of the dose-error quantity versus values of spatial-error quantities for a given region of interest.
Fig. 6 shows an example of another informative table displayed by a display unit used in combination with a dose error determination device.
Fig. 7 shows a schematic representation of another embodiment of a dose error determination device.
Fig. 8 shows a schematic representation of another embodiment of a dose error determination device.

DETAILED DESCRIPTION OF EMBODIMENTS

Before turning to a description of the enclosed drawings, some disclosure on considerations underlying the present invention will be given in the following.

In radiotherapy, the statistics of the dose delivered to an ROI is summarized and presented to the user in a graphical form, e.g., dose volume histogram, or informative table containing dose parameters. In order to be able to calculate the dose statistics, the ROI is represented by a set of voxels. For each voxel, the value of the dose at that voxel is interpolated from a 3D dose volume. The uncertainty (due to spatial errors) in estimating the dose at an ROI voxel with coordinates $(x_i, y_i, z_i)$ can be quantified by the difference between the dose d at the position $(x_i, y_i, z_i)$ and the dose d at the "true" voxel position $(\hat{x}_i, \hat{y}_i, \hat{z}_i)$. The difference between $(\hat{x}_i, \hat{y}_i, \hat{z}_i)$ and $(\hat{x}_i, \hat{y}_i, \hat{z}_i)$ is the spatial error vector at voxel $i$

$$\left(e_{x,i}, e_{y,i}, e_{z,i}\right) = (x_i, y_i, z_i) - (\hat{x}_i, \hat{y}_i, \hat{z}_i). \tag{1}$$

In the following, the magnitude of the error vector is denoted by r. For the case in which the spatial errors are due to DIR inaccuracies, determining the "true" voxel position requires manual editing to determine $(\hat{x}_i, \hat{y}_i, \hat{z}_i)$ or, equivalently, it requires the error vector. In clinical practice, however, the true voxel position or the error vector, are not available.

The embodiments of the dose error determination device and method disclosed herein allow calculating in an efficient way the impact of the magnitude of the contour mapping errors on dose parameters. The proposed solution consists of several components, which will be described in more abstract form in the following and elucidated further below more specifically when the description turns to the enclosed drawings:

i) For a user-selected value of magnitude of error r, an output unit such as a graphical user interface (GUI) informs the user on the values of the upper bounds of the maximum dose change for each ROIs. The value of r can be provided by the user as a numeric value and represents a user estimate of the maximum error magnitude for an ROI. In addition, for each ROI the GUI can suggest a range of r values from previous studies on population data.

For example, an upper bound for the dose difference $d(x_i, y_i, z_i)$ - $d(\hat{x}_i, \hat{y}_i, \hat{z}_i)$ can be established by using the mean value theorem. In detail, the mean value theorem for the case of functions with three variables f and with real values, i.e., $f: D \to \mathbb{R}$, where D is a subset of $\mathbb{R}^3$, states that if D is an open and connected set and f is continuous and differentiable on D then, for any two points $(x_i, y_i, z_i)$ and $(\hat{x}_i, \hat{y}_i, \hat{z}_i)$ such that the line segment L uniting $(x_i, y_i, z_i)$ and $(\hat{x}_i, \hat{y}_i, \hat{z}_i)$ belongs to D, there is a point (a, b, c) E L such that

$$f(x_i, y_i, z_i) - f(\hat{x}_i, \hat{y}_i, \hat{z}_i) = \nabla f(a, b, c) \cdot \left( (x_i, y_i, z_i) - (\hat{x}_i, \hat{y}_i, \hat{z}_i) \right), \quad (2)$$

where $\nabla f(a, b, c) \in \mathbb{R}^3$ is the gradient *of f* at (*a, b, c*) and "." is the scalar product between two vectors.

From the Cauchy-Schwartz's inequality it follows that

$$|f(x_i, y_i, z_i) - f(\hat{x}_i, \hat{y}_i, \hat{z}_i)| \le \|\nabla f(a, b, c)\| \ \|(x_i, y_i, z_i) - (\hat{x}_i, \hat{y}_i, \hat{z}_i)\|, \quad (3)$$

where $\| \ \|$ denotes the Euclidian norm on $\mathbb{R}^3$.

Since the dose distribution d satisfies the conditions of the mean value theorem, by applying inequality (2) it follows that for any point (*x, y, z*) within a sphere centered at ($x_i$, $y_i$, $z_i$) and with radius r that

$$|d(x, y, z) - d(x_i, y_i, z_i)| \le \max_{(x,y,z)} \|\nabla d(x, y, z)\| \, r, \quad (4)$$

Therefore, for an ROI for which (as an example) the maximum DIR uncertainty is smaller than or equal to r, the following inequality holds for the maximum dose change (mDC)

$$\mathrm{mDC} \le \min \left( \max_{(x,y,z) \in \mathrm{ROI\_r}} \|\nabla d(x, y, z)\| \, r, \ \max_{(x,y,z) \in \mathrm{D}} d \right) \quad (5)$$

where ROI r denotes a volume obtained by uniformly expanding the ROI volume with r and D is the dose grid. Here we denote the right hand side of inequality (5) by "upper bound" (UB).

Note that inequality (5) holds not only for spatial errors that can be described as displaced voxel position (e.g., from the true position ($\hat{x}_i$, $\hat{y}_i$, $\hat{z}_i$) to position ($x_i$, $y_i$, $z_i$) but also for spatial errors that result in missing or extra voxels up to a distance r from the boundary of the "true" ROI.

The following description now turns to the enclosed drawings.

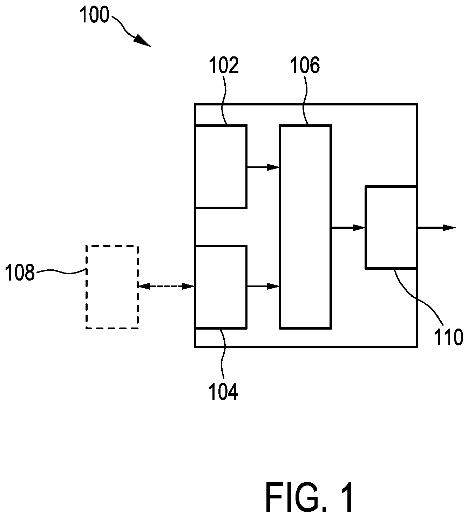

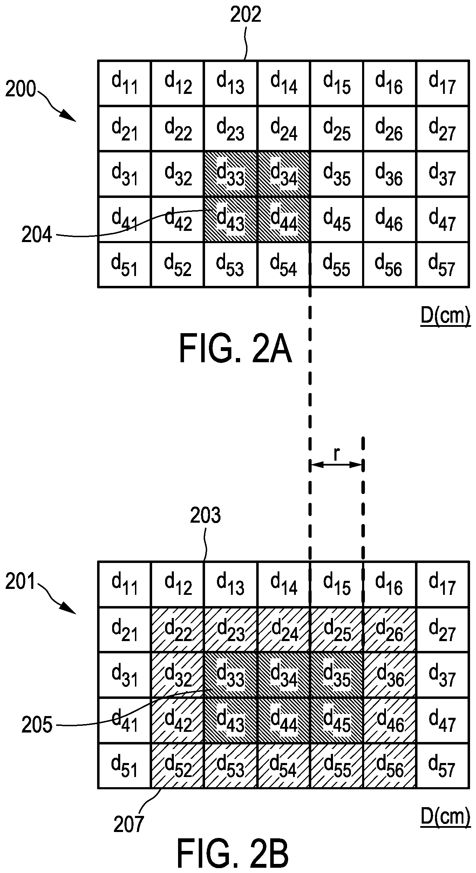

Fig. 1 shows a schematic representation of an embodiment of a dose error determination device 100 for forecasting radiation dose error for a body volume comprising one or more regions of interest subject to radiation treatment based on a dose plan. The explanation of the dose error determination device will be done with further reference to Figs. 2A and 2B which show a first image 200 and a second image 201 of the same body volume. The images show a respective region of interest 204, 205, which is associated to a different set of voxels. Each voxel is additionally associated to respective dose value $d_{xy}$ in accordance with a dose plan. The dose error determination device comprises a dose plan unit 102 configured to provide dose plan data indicative of dose values associated with voxels of a current image of the body volume 200 comprising voxels ($d_{33}$, $d_{34}$, $d_{43}$, $d_{44}$) associated with the region of interest (ROI) 204. The dose error determination device 100 also comprises an input unit 104, that is configured to request and receive user input indicative of a value of a spatial-error quantity corresponding to an amount of a spatial error of voxel coordinates in the current image associated with corresponding features of the body volume. For instance, a use who has access to the first image 200 and to the second image 201 of the body volume identifies that between the ROI 204 and the ROI 500, there is a spatial error or misalignment. The user then can manually input using the input unit the value of the value of a spatial-error quantity, which is r in the case of Figs. 2A and 2B, as a numerical value. Additionally, some dose error determination devices comprise a statistics unit 108 that stores statistical population data associating, based on a statistical population of previous measurements, statistical values associated with the spatial-error quantity to respective regions of interest. The input unit is in these particular dose error determination devices configured to request and receive user input indicative of at least one region of interest and, in response to receiving such user input, to access the statistics unit and to ascertain the statistical values associated with the spatial-error quantity for those regions of interest indicated by the user input, and to subsequently provide the request for user input indicative of the value of the spatial-error quantity in association with an output indicative of the ascertained respective statistical value.

Regardless of whether the dose error determination device includes a statistics unit or not, it comprises a dose-error determining unit 106 that is configured to receive the dose plan data and the value of the spatial error quantity r and to generate dose variation data indicative of a rate of change of the dose values $d_{xy}$ in a respective modified region of interest 207 that in comparison with the corresponding region of interest 205 is expanded in volume by an amount corresponding with the value of the spatial-error quantity r. The dose-error determining unit is also configured to determine and provide a respective value of a dose-error quantity associated to the region of interest. This is done by using a calculation rule that represents a positive correlation of the dose-error quantity with the determined dose variation data. This calculation rule involves in some particular dose-determination unit a multiplication operation between the dose variation data for each voxel with the value of the spatial-error quantity. For instance, in some dose error determination devices in accordance with the present invention, the error determination unit is configured to determine, for each voxel of the modified region of interest, local dose variation data representing a product of an amount of a local rate of change of the current dose values associated with the given voxel and of the value of the spatial-error quantity, and to determine the dose variation data associated with the region of interest as a maximum amount of the product within the modified region of interest. Other calculation rules comprise a multiplication of the dose variation data with values obtained using a monotonically increasing function, so that for increasing values of the dose variation data, the resulting dose-error quantity does not decrease. In some advantageous dose error determination devices and independently on the calculation rule used, the dose-error determining unit is further configured to determine as the dose variation quantity a maximum dose value and a minimum dose value among those dose values corresponding to voxels located within a distance given by the spatial-error quantity with respect to any voxel associated with each region of interest.

The dose error determination device also comprises an output unit 110 which receives from the dose-error determination unit the respective value of the dose-error quantity of the one or more ROIs and is configured to provide an output indicative thereof.

Advantageously, the dose error determination unit is used in combination with a display unit that is configured to receive from the output unit 110 the output indicative of the dose error quantity of the one or more ROIs. The display unit may comprise a graphical user interface configured to display the data received from the output unit in a way which enables the user to focus on the most critical ROIs in dependence on the dose plan and the determined dose-error quantities. Depending on the actual information content of the output, the graphical user interface may be configured to displays the output data in different ways.

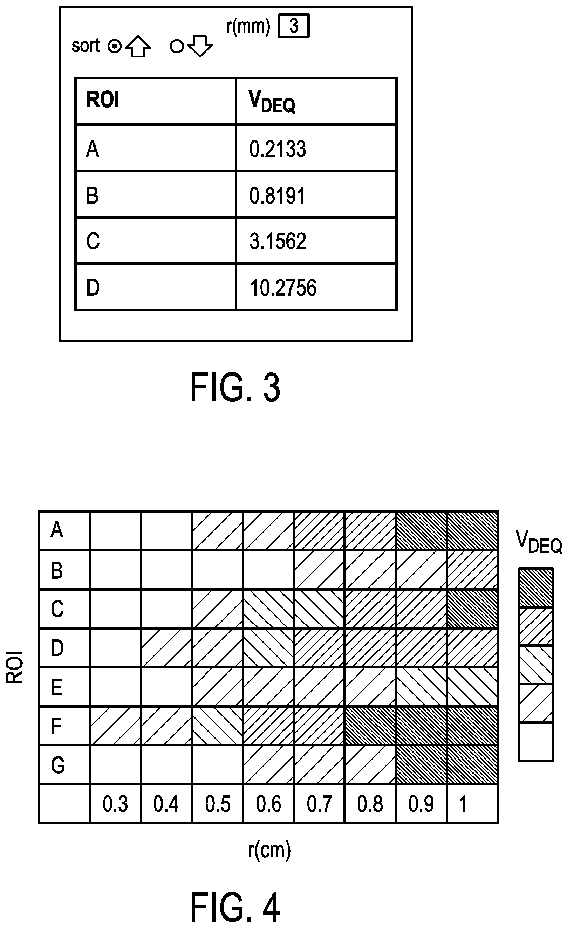

For instance, an exemplary graphical user interface that can be used in combination with any of the dose error determination devices in accordance with the invention is configured to display the output data in the form of an informative table, listing the one or more ROIs in a predetermined order, such as in an increasing or decreasing order of the associated dose-error quantity. An example of an informative table displayed at the displayed unit is shown in Fig. 3. The figure shows an informative table presenting a list of ROIs A, B, C and D together with their respective determined dose-error quantities $V_{DEQ}$ for a given value of the spatial error quantity, which is this particular case is 3mm.

A user can select how the list is to be sorted, i.e. in an increasing or a decreasing order of the determined dose-error quantities, which are indicative of the maximum dose change expected at the ROI for the given value of the spatial error quantity. By providing such a table, the user might decide to skip manual editing for the ROIs for which, for example, the estimated impact of contour inaccuracies given by the value of the spatial error quantity on the dose parameters is small. Similarly, the user could choose to manually review the ROIs for which small contour mapping errors result in large dose changes, as given by the associated value of the dose-error quantity. Note that the value of the dose-error quantity is a measure of sensitivity for all voxels of a given ROI, and not only for the voxels with maximum dose.

In the case of dose error determination devices that include an input unit further configured to request and receive dose-tolerance user input indicative of a tolerance interval of the dose-error quantity associated with a given region of interest, the user can provide thresholds or ranges for accepted values of maximum dose changes (i.e. values of dose-error quantities). In an informative table such as that of Fig. 3, the ROIs and their corresponding values of dose-error quantities can be advantageously provided in accordance with a predetermined color coding, such as for instance a traffic light color coding with red yellow and green colors.

Some dose error determination devices are suitably configured to determine the values of dose-error quantity for one or more ROIs and for a plurality of values of the spatial-error quantity. These dose error determination devices can be advantageously used in combination with a display unit configured to generate a sensitivity map, such as the one shown in Fig. 4. The sensitivity map of Fig. 4 includes a listing of ROIs, A-G, in a vertical axis and a sequence of values of the spatial-error quantity, in this case a magnitude of spatial error due to inaccuracies in a deformable image registration, in a horizontal axis. The dose error determination device determines the for each ROI and for each value of the spatial error quantity the corresponding value of the dose-error quantity. The display unit is then advantageously configured to display the output data as a sensitivity map using a predetermined color coding, wherein in color represents a range of the values of dose error quantity $V_{DEQ}$.

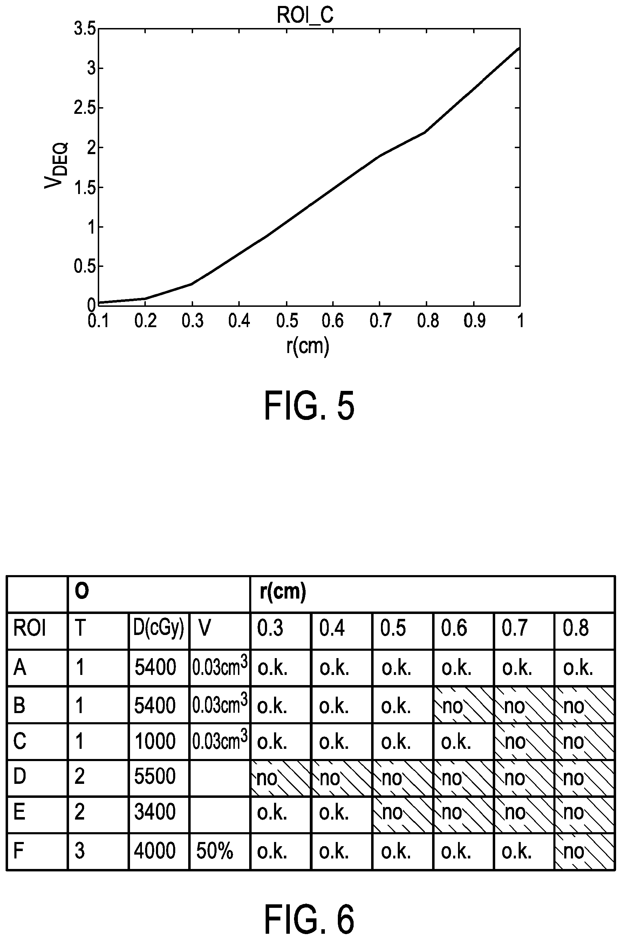

Another example of the display unit can be alternatively or additionally configured to display the output data as a graph plotting, for one or more ROIs, the value of the dose-error quantity, for example in a vertical axis, versus the value of the spatial error quantity in the horizontal axis, as it is exemplarily shown in Fig. 5.

Dose error determination devices that are configured to determine as the dose variation quantity a maximum dose value and a minimum dose value among those dose values corresponding to voxels located within a distance given by the spatial-error quantity with respect to any voxel associated with each region of interest can be advantageously used in combination with a display unit configured to display an upper and a lower bound dose volume histogram (DVH) for a given ROI and a given value of the spatial-error quantity. Additionally, upper and lower bounds of dose value

parameters inferred from the dose plan such as a mean dose value for a given ROI or a value of a fractional volume indicative of that volume fraction for which an estimated received radiation is higher than a predetermined radiation threshold, can be provided also in the form of an informative table.

Some display units are also configured to display an interactive graph that allows the user to interactively inspect all values of the dose-error quantity for a specific value of the spatial-error quantity. For instance, such a display unit can be configured to provide the value of the dose-error quantity for a given value of the spatial-error quantity, or alternatively or additionally, a maximum value of tolerated spatial-error quantity such that the value of the spatial-error quantity satisfies a predetermined constrain, such as a maximum allowed value thereof. These displays units are advantageously used with dose error determination devices that include a dose-objective input interface for receiving dose-objective data for the region of interest indicative of desired values of dose-objective quantities associated with the dose plan. In these dose error determination devices, the dose-error determining unit is additionally configured, using the dose-plan data and the determined value of the dose-error quantity for the region of interest, to generate dose-forecast data for the region of interest indicative of forecast values of the dose-objective quantities; and the output unit is configured to provide the output as additionally being indicative of the dose-objective data and of the dose-forecast data.

Another example of a graphical user interface of a display unit that can be used in combination with a dose error determination device in accordance with the present invention is shown in Fig. 6. The table shown in Fig. 6 includes a first column with the list of the ROIs, A-F. It also shows the objectives O for each ROI. The objectives include the information pertaining to a type of value of dose-error quantity (e.g. 1 being a maximum value of DVH, 2 being a mean dose value, 3 being a percentage of the maximum DVH), to the desired dose value D in accordance with the dose plan and to a volume value V, either in terms of absolute volume or relative volume. The list of objectives is may be automatically imported from a treatment plan in Pinnacle, or the user could manually fill in the objectives. For each ROI, the graphical user interface will indicate whether for a certain range of DIR errors, also given in the table of Fig. 6 as r(cm), the objectives are met (ok), or not met (no). The user could decide to skip review of ROIs that are robust to a large range of DIR errors and instead review the ROIs that are sensitive to DIR uncertainties. The names of ROIs that are relatively more sensitive to spatial errors could be highlighted by, for instance displaying them in a larger font, or displaying them in a different font color, or intermittently displaying them with a blinking pattern, etc.)

Yet another display unit is advantageously configured to enable a robustness evaluation of the dose parameters against values of spatial-error quantities such as DIR uncertainties by applying a user-selected model of uncertainties by means of a dedicated graphical user interface. A model of uncertainty is a parametric model that modifies the physical coordinates of the ROI's voxels, thus producing a second instance of the ROI. Several uncertainty models can be provided, such as for example, worst case scenario DVH, rotations around the three axis, translations, shrinking or expanding the ROI with margins, combination of translations with rotations, random perturbations of the ROI surface, parametric models of errors learned from previous data using principal component analysis, or any combination of the previously mentioned models. The user could select the desired uncertainty model from a drop-down menu. For each uncertainty model, the user can input parameter values or select a range of values using for instance a slider. The value of the dose-error quantity can be calculated for each selected uncertainty model and value(s) of spatial-error quantity. The results of the robustness evaluation will be presented, for example, in the form of a figure displaying the dose volume histograms corresponding to the selected uncertainty model, and baseline DVH (e.g., before applying the DIR error). The user can also be presented with a table summarizing, for instance, dosimetric objectives for each selected ROI, or baseline value of the objective or, the objective value for the selected value of DIR error, or any combination thereof. A traffic light system can for example be used to notify the user whether the objective for the selected DIR error value is met (green), equal (yellow), or not met (red).

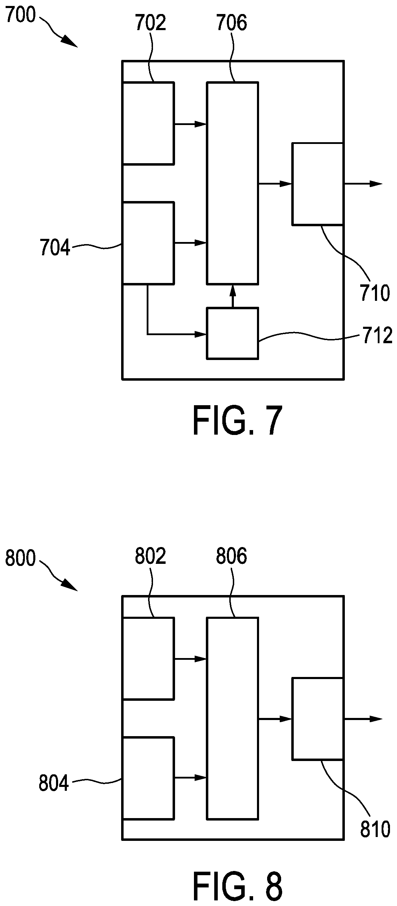

This display unit is advantageously used in combination with a dose error determination device such as the dose error determination device 700 shown in Fig. 7. The dose error determination device 700 comprises a ROI modelling unit 712, which is configured to generate, in response to a selection instruction received via the input unit 704, second instance region of interest data associated with a given one of the one or more regions of interest by modifying, in accordance with a selected one of a set of one or more respective uncertainty models defining spatial coordinate transformation instructions for transforming the region of interest only in regard to its spatial voxel coordinates, thus providing a representation of a transformed region of interest. In the dose error determination device 700, the dose-error determining unit 706 is additionally configured to determine and provide an expected value of the dose-error quantity associated with the transformed region of interest, and the input unit 704 is additionally configured to request and receive user input indicative of the uncertainty model to be used by the ROI modelling unit.

Yet another display unit is advantageously configured to offer the user a sensitivity tool configured to analyze plan robustness to spatial errors, such as, but not limited to, ROI contouring errors or geometric image artifacts. This display unit can advantageously be used to compare rival radiation plans for a patient and thus assist in a choice of radiation plan. This analysis can be performed manually by the expert or in automated mode. Furthermore, the results of such sensitivity analysis could be taken as an input in a further optimization process to generate plans robust to contouring errors. For instance, two different radiation plans A and B for a given patient P can be compared in terms of robustness to spatial errors of an ROI in the following way: for each plan A and B, a plurality of values of the spatial-error quantity are applied and the corresponding value of the dose-error quantity associated to plan A and plan B is calculated. The value of the dose-error quantity is in a particular example a standard deviation of a fractional ROI volume receiving dose higher than a threshold dose value v. In another example it is indicative of the width of the DVH band a certain value of the dose The width of the DVH bands for two plans can be shown to the user as numerical quantities or displayed in a graphical way.

This display unit is advantageously used in combination with a dose error determination device such as the dose error determination device 800 shown in Fig. 8. The dose error determination device 800 comprises a dose plan unit 802 that is configured to provide a plurality of dose plans, each comprising respective dose plan data associated with alternative dose plans for the region of interest. The dose-error determination unit 806 is further configured to provide plan-robustness data indicative of a respective plan-robustness value for each dose plan and in dependence of the received value of the spatial-error quantity, the plan-robustness value being determined in accordance with a predetermined rule based on the determined values of the dose-error quantities associated with the one or more regions of interest. Also, the output unit 810 is further configured to receive the plan-robustness data and to provide an output indicative thereof.

In summary, a dose error determination device for forecasting radiation dose error for a body volume comprising one or more regions of interest subject to radiation treatment based on a dose plan in presented. The dose error determination device comprises a dose-error determining unit configured to generate, using dose plan data for the respective region of interest and an input value of the spatial-error quantity, dose variation data indicative of a rate of change of the dose values in a respective modified region of interest that in comparison with the respective region of interest is expanded in volume by an amount corresponding with the value of the spatial-error quantity and to determine and provide a respective value of a dose-error quantity associated with the respective region of interest, using a calculation rule representing a positive correlation of the dose-error quantity with the determined dose variation data.

In the claims, the word "comprising" does not exclude other elements or steps, and the indefinite article "a" or "an" does not exclude a plurality.

A single step or other units may fulfill the functions of several items recited in the claims. The mere fact that certain measures are recited in mutually different dependent claims does not indicate that a combination of these measures cannot be used to ad-vantage.

Any reference signs in the claims should not be construed as limiting the scope.

## Claims

1. A dose error determination device (100) for forecasting radiation dose error for a body volume comprising one or more regions of interest subject to radiation treatment based on a dose plan, the dose error determination device comprising:

   - a dose plan (102) unit configured to provide dose plan data indicative of dose values associated with voxels of a current image of the body volume comprising voxels associated with the region of interest;
   - a dose-error determining unit (106), which is configured to generate, using the dose plan data for the respective region of interest and a value of a spatial-error quantity, dose variation data for each voxel; and **characterized by**
   - an input unit (104) configured to request and receive user input indicative of the value of a spatial-error quantity corresponding to an amount of a spatial error of voxel coordinates in the current image associated with corresponding features of the body volume;
   - and by the dose-error determining unit (106), being configured: :
   - to generate, using the dose plan data for the respective region of interest and the value of the spatial-error quantity, dose variation data indicative of a rate of change of the dose values in a respective modified region of interest that in comparison with the respective region of interest is expanded in volume by an amount corresponding with the value of the spatial-error quantity; and
   - to determine and provide a respective value of a dose-error quantity associated with the respective region of interest, using a calculation rule representing a positive correlation of the dose-error quantity with the determined dose variation data; and by
   - an output unit (110), which receives the respective value of the dose-error quantity, and is configured to provide an output indicative thereof.

2. The dose error determination device of claim 1, wherein the dose-error determining unit is configured to determine, for each voxel of the modified region of interest, local dose variation data representing a product of an amount of a local rate of change of the current dose values associated with the given voxel and of the value of the spatial-error quantity, and to determine the dose variation data associated with the region of interest as a maximum amount of the product within the modified region of interest.

3. The dose error determination device of claim 2, wherein the dose-error determining unit is further configured to identify a maximum dose value among the dose plan data, and, upon determining that the identified maximum dose value is smaller in amount than the determined maximum of the product within the modified region of interest, to provide to the output unit the identified maximum dose value as the respective value of the dose-error quantity associated with the respective region of interest.

4. The dose error determination device of claim 1, wherein the dose-error determining unit is further configured to determine as the dose variation quantity a maximum dose value and a minimum dose value among those dose values corresponding to voxels located within a distance given by the spatial-error quantity with respect to any voxel associated with each region of interest.

5. The dose error determination device of claim 1, wherein

- the input unit is further configured to request and receive dose-tolerance user input indicative of a tolerance interval of the dose-error quantity associated with a given region of interest;
- the dose-error determining unit is further configured:
- to receive the tolerance interval; and
- to determine, additionally using the tolerance interval, a relation between the determined value of the dose-error quantity and the tolerance interval associated with the given region of interest; and wherein
- the output unit is configured to provide the output as additionally being indicative of the determined relation.

6. The dose error determination device of claim 1,

- further comprising a statistics unit (108) storing statistical population data associating, based on a statistical population of previous measurements, statistical values associated with the spatial-error quantity to respective regions of interest; wherein
- the input unit is configured to request and receive user input indicative of at least one region of interest and, in response to receiving such user input, to access the statistics unit and to ascertain the statistical values associated with the spatial-error quantity for those regions of interest indicated by the user input, and to subsequently provide the request for user input indicative of the value of the spatial-error quantity in association with an output indicative of the ascertained respective statistical value.

7. The dose error determination device of claim 1, further comprising

- a dose-objective input interface for receiving dose-objective data for the region of interest indicative of desired values of dose-objective quantities associated with the previous dose plan; wherein
- the dose-error determining unit is additionally configured, using the dose-plan data and the determined value of the dose-error quantity for the region of interest, to generate dose-forecast data for the region of interest indicative of forecast values of the dose-objective quantities; and wherein
- the output unit is configured to provide the output as additionally being indicative of the dose-objective data and of the dose-forecast data.

8. The dose error determination device of claim 1, further comprising

- a ROI modelling unit, which is configured to generate, in response to a selection instruction received via the input unit, second instance region of interest data associated with a given one of the one or more regions of interest by modifying, in accordance with a selected one of a set of one or more respective uncertainty models defining spatial coordinate transformation instructions for transforming the region of interest only in regard to its spatial voxel coordinates, thus providing a representation of a transformed region of interest;
- wherein the dose-error determining unit is additionally configured to determine and provide an expected value of the dose-error quantity associated with the transformed region of interest; and
- wherein the input unit is additionally configured to request and receive user input indicative of the uncertainty model to be used by the ROI modelling unit.

9. The dose error determination device of claim 1, wherein

- the output unit is configured to associate a color-code value to the received value of the dose-error quantity, in accordance with a predetermined color code assigning different colors to different predetermined value intervals of the dose-error quantity, and to generate and provide the output as additionally being indicative of the color assigned to the respective value of the dose-error quantity.

**10.** The dose error determination device of claim 8, wherein

- the output unit is additionally configured to receive two-dimensional image data representing an image of the respective region of interest, and to generate and provide the output of the dose-error quantity in the form of a graphical overlay onto the image of the respective region of interest.

**11.** The dose error determination device of claim 1, wherein:

- the dose plan unit is configured to provide a plurality of dose plans, each comprising respective dose plan data associated with alternative dose plans for the region of interest; wherein
- the dose-error determining unit is further configured to provide plan-robustness data indicative of a respective plan-robustness value for each dose plan and in dependence of the received value of the spatial-error quantity, the plan-robustness value being determined in accordance with a predetermined rule based on the determined values of the dose-error quantities associated with the one or more regions of interest; and wherein
- the output unit is further configured to receive the plan-robustness data and to provide an output indicative thereof.

**12.** A computer-implemented dose error determination method for forecasting radiation dose error for a body volume comprising one or more regions of interest subject to radiation treatment based on a dose plan, the dose error determination method comprising:

- providing dose plan data indicative of dose values associated with voxels of a current image of the body volume comprising voxels associated with the region of interest; the method being **characterized by** further comprising the steps of
- requesting and receiving user input indicative of a value of a spatial-error quantity corresponding to an amount of a spatial error of voxel coordinates in the current image associated with corresponding features of the body volume;
- generating, using the dose plan data for the respective region of interest and the value of the spatial-error quantity, dose variation data indicative of a rate of change of the dose values in a respective modified region of interest that in comparison with the respective region of interest is expanded in volume by an amount corresponding with the value of the spatial-error quantity;
- determining and providing a respective value of a dose-error quantity associated with the respective region of interest, using a calculation rule representing a positive correlation of the dose-error quantity with the determined dose variation data; and
- providing an output indicative the respective value of the dose-error quantity.

**13.** A computer program comprising instructions which, when the program is executed by a computer, cause the computer to carry out the method of claim 12.

**Patentansprüche**

**1.** Dosisfehlerbestimmungsvorrichtung (100) zum Vorhersagen eines Strahlendosisfehlers für ein Körpervolumen, das einen oder mehrere interessierende Bereiche umfasst, die einer Strahlenbehandlung basierend auf einem Dosisplan unterzogen werden, wobei die Dosisfehlerbestimmungsvorrichtung umfasst:

- eine Dosisplan-Einheit (102), die konfiguriert ist, um Dosisplandaten bereitzustellen, die Dosiswerte anzeigen, die Voxeln eines aktuellen Bildes des Körpervolumens zugeordnet sind, das Voxel umfasst, die mit dem interessierenden Bereich verknüpft sind;
- eine Dosisfehler-Bestimmungseinheit (106), die konfiguriert ist, um unter Verwendung der Dosisplandaten für den jeweiligen interessierenden Bereich und eines Wertes einer räumlichen Fehlergröße Dosisvariationsdaten für jedes Voxel zu erzeugen; und **gekennzeichnet durch**
- eine Eingabeeinheit (104), die konfiguriert ist, um eine Benutzereingabe anzufordern und zu empfangen, die den Wert einer räumlichen Fehlergröße anzeigt, die einer Menge eines räumlichen Fehlers von Voxelkoordinaten in dem aktuellen Bild entspricht, das entsprechenden Merkmalen des Körpervolumens zugeordnet ist;
- und durch die Dosisfehler-Bestimmungseinheit (106), die konfiguriert ist, zum:
- unter Verwendung der Dosisplandaten für den jeweiligen interessierenden Bereich und des Wertes der räumlichen Fehlergröße, Erzeugen von Dosisänderungsdaten, die eine Änderungsrate der Dosiswerte in einem jeweiligen modifizierten interessierenden Bereich anzeigen, der im Vergleich zu dem jeweiligen interessierenden Bereich im Volumen um eine Menge erweitert ist, die dem Wert der räumlichen Fehlergröße entspricht; und
- Bestimmen und Bereitstellen eines jeweiligen Wertes einer Dosisfehlergröße, die dem jeweiligen interessierenden Bereich zugeordnet ist, unter Verwendung einer Berechnungsvorschrift, die eine positive Korrelation der Dosisfehlergröße mit den ermittelten Dosisvariationsdaten darstellt; und durch
- eine Ausgabeeinheit (110), die den jeweiligen Wert der Dosisfehlergröße empfängt und konfiguriert ist, um eine diese anzeigende Ausgabe bereitzustellen.

2. Dosisfehlerbestimmungsvorrichtung nach Anspruch 1, wobei die Dosisfehler-Bestimmungseinheit konfiguriert ist, um für jedes Voxel des modifizierten interessierenden Bereichs lokale Dosisänderungsdaten zu bestimmen, die ein Produkt einer Menge einer lokalen Änderungsrate der dem gegebenen Voxel zugeordneten aktuellen Dosiswerte und des Wertes der räumlichen Fehlergröße darstellen, und die Dosisänderungsdaten, die dem interessierenden Bereich zugeordnet sind, als maximale Produktmenge innerhalb des modifizierten interessierenden Bereichs zu bestimmen.

3. Dosisfehlerbestimmungsvorrichtung nach Anspruch 2, wobei die Dosisfehler-Bestimmungseinheit ferner konfiguriert ist, um einen maximalen Dosiswert unter den Dosisplandaten zu identifizieren, und, wenn bestimmt wird, dass der identifizierte maximale Dosiswert betragsmäßig kleiner ist als das ermittelte Maximum des Produkts innerhalb des modifizierten interessierenden Bereichs, Bereitstellen des identifizierten maximalen Dosiswerts als den jeweiligen Wert der Dosisfehlergröße, die dem jeweiligen interessierenden Bereich zugeordnet ist, an die Ausgabeeinheit.

4. Dosisfehlerbestimmungsvorrichtung nach Anspruch 1, wobei die Dosisfehler-Bestimmungseinheit ferner konfiguriert ist, um als Dosisvariationsgröße einen maximalen Dosiswert und einen minimalen Dosiswert unter jenen Dosiswerten zu bestimmen, die Voxeln entsprechen, die sich innerhalb eines Abstands befinden, der durch die räumliche Fehlergröße in Bezug auf jedes Voxel gegeben ist, das jedem interessierenden Bereich zugeordnet ist.

5. Dosisfehlerbestimmungsvorrichtung nach Anspruch 1, wobei

- die Eingabeeinheit ferner konfiguriert ist, um eine Dosistoleranz-Benutzereingabe anzufordern und zu empfangen, die ein Toleranzintervall der Dosisfehlergröße anzeigt, das einem gegebenen interessierenden Bereich zugeordnet ist;
- wobei die Dosisfehler-Bestimmungseinheit ferner konfiguriert ist, zum:
- Empfangen des Toleranzintervalls; und
- zusätzlich unter Verwendung des Toleranzintervalls, Bestimmen einer Beziehung zwischen dem bestimmten Wert der Dosisfehlergröße und dem Toleranzintervall, das dem gegebenen interessierenden Bereich zugeordnet ist; und wobei
- die Ausgabeeinheit konfiguriert ist, um die Ausgabe bereitzustellen, die zusätzlich die bestimmte Beziehung anzeigt.

6. Dosisfehlerbestimmungsvorrichtung nach Anspruch 1,

- ferner umfassend eine Statistikeinheit (108), die statistische Populationsdaten speichert, die basierend auf einer statistischen Population von vorherigen Messungen statistische Werte, die der räumlichen Fehlergröße zugeordnet sind, jeweiligen interessierenden Bereichen zuordnen; wobei
- die Eingabeeinheit konfiguriert ist, um Benutzereingaben anzufordern und zu empfangen, die mindestens einen interessierenden Bereich anzeigen, und als Reaktion auf den Empfang einer solchen Benutzereingabe auf die Statistikeinheit zuzugreifen und die statistischen Werte zu ermitteln, die der räumlichen Fehlergröße für die durch die Benutzereingabe angezeigten interessierenden Bereiche zugeordnet sind, und nachfolgend die Aufforderung zur Benutzereingabe bereitzustellen, die den Wert der räumlichen Fehlergröße anzeigt, in Verbindung mit einer Ausgabe, die den ermittelten jeweiligen statistischen Wert anzeigt.

7. Dosisfehlerbestimmungsvorrichtung nach Anspruch 1, ferner umfassend:

- eine Dosisziel-Eingabeschnittstelle zum Empfangen von Dosiszieldaten für den interessierenden Bereich, die die gewünschten Werte von Dosisziel-Größen anzeigen, die dem vorherigen Dosisplan zugeordnet sind; wobei
- die Dosisfehler-Bestimmungseinheit zusätzlich dazu konfiguriert ist, unter Verwendung der Dosisplandaten und des ermittelten Wertes der Dosisfehlergröße für den interessierenden Bereich Dosisvorhersagedaten für den interessierenden Bereich zu erzeugen, die Vorhersagewerte der Dosiszielgrößen anzeigen; und wobei
- die Ausgabeeinheit konfiguriert ist, um die Ausgabe bereitzustellen, die zusätzlich die Dosiszieldaten und die Dosisvorhersagedaten anzeigt.

8. Dosisfehlerbestimmungsvorrichtung nach Anspruch 1, ferner umfassend:

- eine ROI-Modellierungseinheit, die konfiguriert ist, um als Reaktion auf eine über die Eingabeeinheit empfangene Auswahlanweisung eine zweite Instanz von Daten des interessierenden Bereichs zu erzeugen, die einer gegebenen der einen oder mehreren interessierenden Bereichen zugeordnet sind, durch Modifizieren von Anweisungen zum Transformieren des interessierenden Bereichs nur in Bezug auf seine räumlichen Voxelkoordinaten gemäß einem ausgewählten aus einem Satz von einem oder mehreren jeweiligen Unsicherheitsmodellen, die die räumliche Koordinatentransformation definieren, wodurch eine Darstellung eines transformierten interessierenden Bereichs bereitgestellt wird;
- wobei die Dosisfehler-Bestimmungseinheit zusätzlich konfiguriert ist, um einen erwarteten Wert der Dosisfehlergröße, die dem transformierten interessierenden Bereich zugeordnet ist, zu bestimmen und bereitzustellen; und
- wobei die Eingabeeinheit zusätzlich konfiguriert ist, um eine Benutzereingabe anzufordern und zu empfangen, die das von der ROI-Modellierungseinheit zu verwendende Unsicherheitsmodell anzeigt.

9. Dosisfehlerbestimmungsvorrichtung nach Anspruch 1, wobei

- die Ausgabeeinheit konfiguriert ist, um dem empfangenen Wert der Dosisfehlergröße einen Farbcodewert gemäß einem vorbestimmten Farbcode zuzuordnen, der verschiedenen vorbestimmten Wertintervallen der Dosisfehlergröße unterschiedliche Farben zuordnet, und die Ausgabe zu erzeugen und bereitzustellen, die zusätzlich die Farbe anzeigt, die dem jeweiligen Wert der Dosisfehlergröße zugeordnet ist.

10. Dosisfehlerbestimmungsvorrichtung nach Anspruch 8, wobei

- die Ausgabeeinheit zusätzlich konfiguriert ist, um zweidimensionale Bilddaten zu empfangen, die ein Bild des jeweiligen interessierenden Bereichs darstellen, und die Ausgabe der Dosisfehlergröße in Form einer grafischen Überlagerung auf das Bild des jeweiligen interessierenden Bereichs zu erzeugen und bereitzustellen.

11. Dosisfehlerbestimmungsvorrichtung nach Anspruch 1, wobei:

- die Dosisplaneinheit so konfiguriert ist, dass sie mehrere Dosispläne bereitstellt, von denen jeder jeweilige Dosisplandaten umfasst, die alternativen Dosisplänen für den interessierenden Bereich zugeordnet sind; wobei
- die Dosisfehler-Bestimmungseinheit ferner konfiguriert ist, um Plan-Robustheitsdaten bereitzustellen, die einen jeweiligen Plan-Robustheitswert für jeden Dosisplan anzeigen, und in Abhängigkeit von dem empfangenen Wert der räumlichen Fehlergröße, wobei der Plan-Robustheitswert gemäß einer vorbestimmten Regel basierend auf den bestimmten Werten der Dosisfehlergrößen bestimmt wird, die dem einen oder den mehreren interessierenden Bereichen zugeordnet sind; und wobei
- die Ausgabeeinheit ferner konfiguriert ist, um die Plan-Robustheitsdaten zu empfangen und eine Ausgabe bereitzustellen, die diese anzeigt.

12. Computerimplementiertes Dosisfehlerbestimmungsverfahren zum Vorhersagen eines Strahlendosisfehlers für ein Körpervolumen, das einen oder mehrere interessierende Bereiche umfasst, die einer Strahlenbehandlung basierend auf einem Dosisplan unterzogen werden, wobei das Dosisfehlerbestimmungsverfahren umfasst:

- Bereitstellen von Dosisplandaten, die Dosiswerte anzeigen, die Voxeln eines aktuellen Bildes des Körpervolumens zugeordnet sind, das Voxel umfasst, die mit dem interessierenden Bereich verknüpft sind;
wobei das Verfahren **dadurch gekennzeichnet ist, dass** es ferner die Schritte umfasst, von:
- Anfordern und Empfangen einer Benutzereingabe, die einen Wert einer räumlichen Fehlergröße anzeigt, die einer Menge eines räumlichen Fehlers von Voxelkoordinaten in dem aktuellen Bild entspricht, das entsprechenden Merkmalen des Körpervolumens zugeordnet ist;

- unter Verwendung der Dosisplandaten für den jeweiligen interessierenden Bereich und des Wertes der räumlichen Fehlergröße, Erzeugen von Dosisänderungsdaten, die eine Änderungsrate der Dosiswerte in einem jeweiligen modifizierten interessierenden Bereich anzeigen, der im Vergleich zu den jeweiligen interessierenden Bereich im Volumen um eine Menge erweitert ist, die dem Wert der räumlichen Fehlergröße entspricht;
- Bestimmen und Bereitstellen eines jeweiligen Wertes einer Dosisfehlergröße, die dem jeweiligen interessierenden Bereich zugeordnet ist, unter Verwendung einer Berechnungsvorschrift, die eine positive Korrelation der Dosisfehlergröße mit den bestimmten Dosisvariationsdaten darstellt; und
- Bereitstellen einer Ausgabe, die den jeweiligen Wert der Dosisfehlergröße anzeigt.

**13.** Computerprogramm, das Anweisungen umfasst, die, wenn das Programm von einem Computer ausgeführt wird, den Computer veranlassen, das Verfahren nach Anspruch 12 auszuführen.

## Revendications

**1.** Dispositif de détermination d'erreur de dose (100) pour prévoir une erreur de dose de rayonnement pour un volume corporel comprenant une ou plusieurs régions d'intérêt soumises à un traitement par rayonnement sur la base d'un plan de dose, le dispositif de détermination d'erreur de dose comprenant:

- une unité de plan de dose (102) configurée pour fournir des données de plan de dose indiquant des valeurs de dose associées aux voxels d'une image actuelle du volume corporel comprenant des voxels associés à la région d'intérêt;
- une unité de détermination d'erreur de dose (106), qui est configurée pour générer, en utilisant les données du plan de dose pour la région d'intérêt respective et une valeur d'une quantité d'erreur spatiale, des données de variation de dose pour chaque voxel; et **caractérisé par**
- une unité d'entrée (104) configurée pour demander et recevoir une entrée utilisateur indiquant la valeur d'une quantité d'erreur spatiale correspondant à une quantité d'erreur spatiale de coordonnées de voxels dans l'image actuelle associée à des caractéristiques correspondantes du volume corporel;
- et par l'unité de détermination d'erreur de dose (106), étant configuré:
- pour générer, en utilisant les données du plan de dose pour la région d'intérêt respective et la valeur de la quantité d'erreur spatiale, des données de variation de dose indiquant un taux de changement des valeurs de dose dans une région d'intérêt modifiée respective qui, par rapport à la région d'intérêt respective est agrandie en volume d'une quantité correspondant à la valeur de la quantité d'erreur spatiale; et
- pour déterminer et fournir une valeur respective d'une quantité d'erreur de dose associée à la région d'intérêt respective, en utilisant une règle de calcul représentant une corrélation positive de la quantité d'erreur de dose avec les données de variation de dose déterminées; et par
- une unité de sortie (110), qui reçoit la valeur respective de la quantité d'erreur de dose, et est configurée pour fournir une sortie indiquant celle-ci.

**2.** Dispositif de détermination d'erreur de dose selon la revendication 1, dans lequel l'unité de détermination d'erreur de dose est configurée pour déterminer, pour chaque voxel de la région d'intérêt modifiée, des données de variation de dose locale représentant un produit d'une quantité d'un taux de changement local des valeurs de dose actuelles associées au voxel donné et de la valeur de la quantité d'erreur spatiale, et pour déterminer les données de variation de dose associées à la région d'intérêt en tant que quantité maximale du produit dans la région d'intérêt modifiée.

**3.** Dispositif de détermination d'erreur de dose selon la revendication 2, dans lequel l'unité de détermination d'erreur de dose est en outre configurée pour identifier une valeur de dose maximale parmi les données du plan de dose, et, lors de la détermination que la valeur de dose maximale identifiée est inférieure en quantité à la valeur maximale déterminée du produit à l'intérieur de la région d'intérêt modifiée, pour fournir à l'unité de sortie la valeur de dose maximale identifiée en tant que valeur respective de la quantité d'erreur de dose associée à la région d'intérêt respective.

**4.** Dispositif de détermination d'erreur de dose selon la revendication 1, dans lequel l'unité de détermination d'erreur de dose est en outre configurée pour déterminer comme quantité de variation de dose une valeur de dose maximale et une valeur de dose minimale parmi les valeurs de dose correspondant à des voxels situés à une distance donnée par la quantité d'erreur spatiale par rapport à tout voxel associé à chaque région d'intérêt.

**5.** Dispositif de détermination d'erreur de dose selon la revendication 1, dans lequel

- l'unité d'entrée est en outre configurée pour demander et recevoir une entrée utilisateur de tolérance de dose indiquant un intervalle de tolérance de la quantité d'erreur de dose associée à une région d'intérêt donnée;
- l'unité de détermination d'erreur de dose est en outre configurée:
- pour recevoir l'intervalle de tolérance; et
- pour déterminer, en utilisant en outre l'intervalle de tolérance, une relation entre la valeur déterminée de la quantité d'erreur de dose et l'intervalle de tolérance associé à la région d'intérêt donnée; et où
- l'unité de sortie est configurée pour fournir la sortie comme étant en outre indicative de la relation déterminée.

6. Dispositif de détermination d'erreur de dose selon la revendication 1,

- comprenant en outre une unité statistique (108) stockant des données statistiques de population associant, sur la base d'une population statistique de mesures précédentes, des valeurs statistiques associées à la quantité d'erreur spatiale à des régions d'intérêt respectives; où
- l'unité d'entrée est configurée pour demander et recevoir une entrée utilisateur indiquant au moins une région d'intérêt et, en réponse à la réception d'une telle entrée utilisateur, pour accéder à l'unité de statistiques et pour déterminer les valeurs statistiques associées à la quantité d'erreur spatiale pour les régions d'intérêt indiquées par l'entrée utilisateur, et pour fournir ensuite la demande d'entrée utilisateur indiquant la valeur de la quantité d'erreur spatiale en association avec une sortie indiquant la valeur statistique respective déterminée.

7. Dispositif de détermination d'erreur de dose selon la revendication 1, comprenant en outre

- une interface d'entrée d'objectif de dose pour recevoir des données d'objectif de dose pour la région d'intérêt indiquant des valeurs souhaitées des quantités d'objectif de dose associées au plan de dose précédent; où
- l'unité de détermination d'erreur de dose est en outre configurée, en utilisant les données du plan de dose et la valeur déterminée de la quantité d'erreur de dose pour la région d'intérêt, pour générer des données de prévision de dose pour la région d'intérêt indiquant des valeurs de prévision des quantités d'objectif de dose; et où
- l'unité de sortie est configurée pour fournir la sortie comme étant en outre indicative des données d'objectif de dose et des données de prévision de dose.

8. Dispositif de détermination d'erreur de dose selon la revendication 1, comprenant en outre

- une unité de modélisation ROI, qui est configurée pour générer, en réponse à une instruction de sélection reçue via l'unité d'entrée, des données de région d'intérêt de deuxième instance associées à l'une région donnée de l'une ou des plusieurs régions d'intérêt en modifiant, conformément à l'un modèle sélectionné d'un ensemble d'un ou plusieurs modèles d'incertitude respectifs définissant des instructions de transformation de coordonnées spatiales, pour transformer la région d'intérêt uniquement en ce qui concerne ses coordonnées spatiales de voxels, fournissant ainsi une représentation d'une région d'intérêt transformée;
- où l'unité de détermination d'erreur de dose est en outre configurée pour déterminer et fournir une valeur attendue de la quantité d'erreur de dose associée à la région d'intérêt transformée; et
- où l'unité d'entrée est en outre configurée pour demander et recevoir une entrée utilisateur indiquant le modèle d'incertitude à utiliser par l'unité de modélisation ROI.

9. Dispositif de détermination d'erreur de dose selon la revendication 1, dans lequel

- l'unité de sortie est configurée pour associer une valeur de code de couleur à la valeur reçue de la quantité d'erreur de dose, conformément à un code de couleur prédéterminé attribuant différentes couleurs à différents intervalles de valeurs prédéterminés de la quantité d'erreur de dose, et pour générer et fournir la sortie comme étant en outre indicative de la couleur attribuée à la valeur respective de la quantité d'erreur de dose.

10. Dispositif de détermination d'erreur de dose selon la revendication 8, dans lequel

- l'unité de sortie est en outre configurée pour recevoir des données d'image bidimensionnelle représentant une image de la région d'intérêt respective, et pour générer et fournir la sortie de la quantité d'erreur de dose sous la forme d'une superposition graphique sur l'image de la région d'intérêt respective.

11. Dispositif de détermination d'erreur de dose selon la revendication 1, dans lequel:

- l'unité de plan de dose est configurée pour fournir une pluralité de plans de dose, chacun comprenant des données de plan de dose respectives associées à des plans de dose alternatifs pour la région d'intérêt; où
- l'unité de détermination d'erreur de dose est en outre configurée pour fournir des données de robustesse de plan indiquant une valeur de robustesse de plan respective pour chaque plan de dose et en fonction de la valeur reçue de la quantité d'erreur spatiale, la valeur de robustesse de plan étant déterminée conformément à une règle prédéterminée basée sur les valeurs déterminées des quantités d'erreur de dose associées à l'une ou aux plusieurs régions d'intérêt; et où
- l'unité de sortie est en outre configurée pour recevoir les données de robustesse de plan et pour fournir une sortie indiquant celles-ci.

12. Procédé de détermination d'erreur de dose mise en œuvre par ordinateur pour prévoir une erreur de dose de rayonnement pour un volume corporel comprenant une ou plusieurs régions d'intérêt soumises à un traitement par rayonnement sur la base d'un plan de dose, le procédé de détermination d'erreur de dose consistant à:

- fournir des données de plan de dose indiquant des valeurs de dose associées aux voxels d'une image actuelle du volume corporel comprenant des voxels associés à la région d'intérêt;
le procédé étant **caractérisé en ce qu'**il comprend en outre les étapes consistant à
- demander et recevoir une entrée utilisateur indiquant la valeur d'une quantité d'erreur spatiale correspondant à une quantité d'erreur spatiale de coordonnées de voxels dans l'image actuelle associée à des caractéristiques correspondantes du volume corporel;
- générer, en utilisant les données du plan de dose pour la région d'intérêt respective et la valeur de la quantité d'erreur spatiale, des données de variation de dose indiquant un taux de changement des valeurs de dose dans une région d'intérêt modifiée respective qui, par rapport à la région d'intérêt respective est agrandie en volume d'une quantité correspondant à la valeur de la quantité d'erreur spatiale;
- déterminer et fournir une valeur respective d'une quantité d'erreur de dose associée à la région d'intérêt respective, en utilisant une règle de calcul représentant une corrélation positive de la quantité d'erreur de dose avec les données de variation de dose déterminées; et
- fournir une sortie indiquant la valeur respective de la quantité d'erreur de dose.

13. Programme informatique comprenant des instructions qui, lorsque le programme est exécuté par un ordinateur, amène l'ordinateur à exécuter le procédé de la revendication 12.

FIG. 1

202

| $d_{11}$ | $d_{12}$ | $d_{13}$ | $d_{14}$ | $d_{15}$ | $d_{16}$ | $d_{17}$ |
|---|---|---|---|---|---|---|
| $d_{21}$ | $d_{22}$ | $d_{23}$ | $d_{24}$ | $d_{25}$ | $d_{26}$ | $d_{27}$ |
| $d_{31}$ | $d_{32}$ | $d_{33}$ | $d_{34}$ | $d_{35}$ | $d_{36}$ | $d_{37}$ |
| $d_{41}$ | $d_{42}$ | $d_{43}$ | $d_{44}$ | $d_{45}$ | $d_{46}$ | $d_{47}$ |
| $d_{51}$ | $d_{52}$ | $d_{53}$ | $d_{54}$ | $d_{55}$ | $d_{56}$ | $d_{57}$ |

200

204

D(cm)

# FIG. 2A

r

203

| $d_{11}$ | $d_{12}$ | $d_{13}$ | $d_{14}$ | $d_{15}$ | $d_{16}$ | $d_{17}$ |
|---|---|---|---|---|---|---|
| $d_{21}$ | $d_{22}$ | $d_{23}$ | $d_{24}$ | $d_{25}$ | $d_{26}$ | $d_{27}$ |
| $d_{31}$ | $d_{32}$ | $d_{33}$ | $d_{34}$ | $d_{35}$ | $d_{36}$ | $d_{37}$ |
| $d_{41}$ | $d_{42}$ | $d_{43}$ | $d_{44}$ | $d_{45}$ | $d_{46}$ | $d_{47}$ |
| $d_{51}$ | $d_{52}$ | $d_{53}$ | $d_{54}$ | $d_{55}$ | $d_{56}$ | $d_{57}$ |

201

205

207

D(cm)

# FIG. 2B

sort ⊙⇧  ○⇩       r(mm) ⬚3⬚

| ROI | V<sub>DEQ</sub> |
|-----|------|
| A | 0.2133 |
| B | 0.8191 |
| C | 3.1562 |
| D | 10.2756 |

## FIG. 3

## FIG. 4

FIG. 5

| | O | | | r(cm) | | | | | |
|---|---|---|---|---|---|---|---|---|---|
| ROI | T | D(cGy) | V | 0.3 | 0.4 | 0.5 | 0.6 | 0.7 | 0.8 |
| A | 1 | 5400 | 0.03cm$^3$ | o.k. | o.k. | o.k. | o.k. | o.k. | o.k. |
| B | 1 | 5400 | 0.03cm$^3$ | o.k. | o.k. | o.k. | no | no | no |
| C | 1 | 1000 | 0.03cm$^3$ | o.k. | o.k. | o.k. | o.k. | no | no |
| D | 2 | 5500 | | no | no | no | no | no | no |
| E | 2 | 3400 | | o.k. | o.k. | no | no | no | no |
| F | 3 | 4000 | 50% | o.k. | o.k. | o.k. | o.k. | o.k. | no |

FIG. 6

700

702    706

704

710

712

# FIG. 7

800

802    806

804

810

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NEIL KIRBY et al.** An automated deformable image registration evaluation of confidence tool. *Physics in Medicine and Biology,* 2016, vol. 61, N203-N214